# EUROPEAN PATENT APPLICATION

(11) **EP 4 838 535 A1**
(43) Date of publication of application: **11.11.2026**
(21) Application number: 26175839.5
(22) Date of filing: 30.04.2026
(51) Int. Cl.: G16H 10/20, G16H 10/60, G16H 50/70, G16H 50/20

(54) **SYSTEM AND METHOD FOR MATCHING SUBJECTS AND CLINICAL TRIALS**

(30) Priority: 08.05.2025 US 202563802009 P
(71) Applicant: Codex Genetics Limited, Shatin New Territories (HK)
(72) Inventor: TSE, Shing Hei, Shatin (HK); YIM, Aldrin Kay Yuen, Shatin (HK); YU, Chi Shing, Shatin (HK); CHAN, Po Shing, Shatin (HK); JIN, Na Na, Shatin (HK)
(74) Representative: Haseltine Lake Kempner LLP

(57) **Abstract**

A system and method matching a subject with suitable clinical trials is disclosed. A receiving module receives (i) a patient profile; and (II) a plurality of clinical trials. An evaluation framework module comprises at least two evaluation agents, with each agent having a specific analysis domain different from the other; and uses a large language model. Each evaluation agent extracts parameters according to the analysis domain and calculates a score indicative of the match. A ranked list of potential suitable clinical trials is generated. The system further comprises a consensus module to resolve discrepancies between ranked trial lists to determine a refined list comprising one or more suitable clinical trials for the patient profile. A feedback module is configured to output the refined list of clinical trials for the patient profile and to receive data indicative of the suitability of the subject to a specific clinical trial.

## Description

### FIELD OF THE DISCLOSURE

The present disclosure relates to a system and method for matching a subject with one or more suitable clinical trials from a plurality of clinical trials, whilst managing access to sensitive information, more particularly medical information of that subject.

### BACKGROUND OF THE DISCLOSURE

Medical information (including genomic data, demographic information, social and behavioural health determinants, phenotypic information at molecular /clinical level) may be collected for patient care. The primary role of this information is to inform the care and treatment provided to an individual patient.

Patient information may be also used to determine the suitability of a particular patient for clinical trial where there may be a lack of established treatment approaches. Unfortunately, patient information is often fragmented across consultation notes, pathology reports, biomarker results, imaging summaries, and treatment histories; and access to this information can be constrained by patient confidentiality considerations.

One specific application in which developing new approaches to treatment is of critical importance is in oncology referral studies; where eligibility depends on integrated review of molecular biomarkers, tumor histology, disease stage, prior treatment exposure, treatment resistance, and performance status, and does not rely on diagnosis alone. Determination of appropriate clinical trials for such cases is problematic in view of the dispersed information, confidentiality issues and the like, leading to difficulties in matching patients and clinical trials.

In many cases with appropriate consent, a de-identified form of such information may in turn be provided for biomedical research. Such information is particularly important for developing new approaches to treatment, including by leveraging data analytics of a population across multiple data repositories which may be in different jurisdictions.

A number of different approaches have been undertaken in providing access to the typically de-identified information collected from patients. Such approaches range from providing access to third parties without any constraints (e.g. one thousand genome database); to provision of access limited only to authenticated researchers (e.g. one hundred thousand genomes database). Furthermore, in an era of increased sensitivity about data privacy; enhanced legislation (such as GDPR (EU), HIPAA (US)) places increased obligation on users, and parties handling such information.

Unfortunately, even where access to information is limited by security and access protocols; there exists a real possibility that malevolent third parties may access individual information from; and re-identify specific patients. It would be appreciated that this risk would mean compromising the privacy of that individual. In one study researchers were able to re-identify individuals from a data set of whole genome sequences by predicting visual traits including eye and skin colour; whilst in another study, certain participants of the one thousand genome subject were able to be re-identified by inferring surnames from short tandem repeats on the Y chromosome; which in combination with other demographics were linked to identified public resources.

Although various approaches to limiting the access by third parties to this information may be implemented including various approaches to de-identification of individual data, technology based security controls (e.g. encryption, access control and the like), data transformation, provision of data in aggregate only, data obfuscation or synthetic data generation; the risk of potential individual re-identification of an individual from medical information provided may lead to patients withholding consent to use of their data for research purposes.

As such, identifying a potentially eligible patient for one or more clinical trials often requires repeated manual review of heterogeneous documents and direct comparison against protocol criteria. This process can be slow, difficult to standardize, and dependent on local familiarity with both the patient record and the active trial portfolio.

Accordingly, there is a need for an improved system and method for the handling of such information which at least addresses or ameliorates such information or provides a useful choice.

### SUMMARY OF THE DISCLOSURE

Features and advantages of the disclosure will be set forth in the description which follows, and in part will be obvious from the description, or can be learned by practice of the herein disclosed principles. The features and advantages of the disclosure can be realized and obtained by means of the instruments and combinations particularly pointed out in the appended claims.

In accordance with a first aspect of the present disclosure, there is provided a system for matching a subject with one or more suitable clinical trials from a plurality of clinical trials.

The system may comprise a receiving module configured to receive (i) a patient profile of the subject, wherein the patient profile comprises a plurality of different parameters; and (ll) a plurality of clinical trials comprising clinical trial criteria with a plurality of different conditions;
an evaluation framework module for evaluating suitability of a patient profile for one or more of the plurality of clinical trials, the evaluation framework module comprising at least two evaluation agents, wherein each agent has a specific analysis domain different from the other; and each evaluation agent is configured to use a large language model.

Each evaluation agent is configured to extract parameters from the patient profile and conditions of the clinical trial criteria according to the specific analysis domain of that agent and calculate a score indicative of the degree of match therebetween; and generate a ranked list of potential suitable clinical trials according to the score indicative of the degree of match for the specific analysis domain for that evaluation agent and the patient profile.

The system may also comprise a consensus module configured to resolve discrepancies between the ranked trial lists for said patient profile by the at least two evaluation agents to determine a refined list comprising one or more suitable clinical trials by (a) using a weighted voting module if conflicting evaluations exist among the ranked lists of potential suitable clinical trials by prioritising trials with analysis domain results derived by more accurate evaluation agents to determine the refined list of suitable clinical trials for the subject; and (b) if the score indicative of the degree of match of potential suitable clinical trials differs by a predetermined similarity difference, using an arbiter module configured to resolve conflicting evaluations, and generate the refined list of suitable clinical trials for the subject.

A feedback module configured to output the refined list of clinical trials for the patient profile and to receive data indicative of the suitability of the subject to a specific clinical trial may be also provided in the system.

Optionally, the system may further comprise a refinement module configured to dynamically adjust matching metrics of the at least two evaluation agents and the arbiter module based on the feedback data by adjusting weights assigned to specific patient parameters and logical parsing rules specific to each evaluation agent upon receiving data indicative of the suitability of the patient to a specific clinical trial.

Optionally, re-ranking by the arbiter module is performed by determining a custom probability score indicating suitability of at least some of the plurality of clinical trials for that subject.

The potential suitable clinical trials may be filtered by predetermined mandatory exclusion criteria to exclude unsuitable clinical trials before generating the ranked list of the potential suitable clinical trials, wherein the predetermined mandatory exclusion criteria are selected from the group comprising molecular biomarkers, primary diagnosis, gender and age.

Each evaluation agent may be configured to cite supporting text for criteria used in the determination of the evaluation score and in the absence of supporting text, the evaluation agent is configured to exclude that data from the evaluation score until external verification.

Optionally, the specific analysis domains of the evaluation agents may be selected from a group comprising a molecular analysis domain; a disease evaluation domain; a demographic domain and a laboratory test results domain.

Optionally, the arbiter module may comprise a first arbiter module for resolving conflicting evaluations for specific contextual patient parameters and a second arbiter module comprises a large language model configured for resolving conflicting evaluations for specific non-contextual patient profile parameters.

Optionally, in the molecular analysis domain the evaluation framework module may be configured for comparison of molecular patient profile parameters and molecular clinical trial conditions and to provide an indicative score representative of the degree of match therebetween together with supporting reasoning; in the disease evaluation domain the evaluation framework module may be configured for comparison of the disease stage patient profile parameters and the corresponding disease stage conditions of the clinical trials and to provide an indicative score representative of the degree of match therebetween together with supporting reasoning; and in the demographic domain the evaluation framework module may be configured for comparison of patient demographic parameters from the patient profile parameters and the corresponding demographic parameters in conditions of the clinical trial; and to provide an indicative score representative of the degree of match therebetween together with supporting reasoning; and in the laboratory test domain the evaluation framework module may be configured for comparison of the patient laboratory results from the patient profile parameters with corresponding laboratory tests conditions of the clinical trial; and to provide an indicative score representative of the degree of match therebetween together with supporting reasoning.

Optionally, 0 is assigned as the score if the subject is evaluated as ineligible for the clinical trial and 1 is assigned as the score if the patient profile parameters and the conditions of the clinical trial criteria match perfectly or assigning a number between these values according to the degree of evaluated match.

The large language model types may be selected from the group comprising Reasoning large language models, Multimodal models, Traditional large language models, and Agentic large language models.

The data indicating the suitability of a patient profile to a corresponding clinical trial may be selected from a group comprising real-world patient outcomes, expert annotations from clinicians, or simulated historical trial data.

The system may further comprise a deidentification module configured to remove personally identifiable information from patient profiles by zero-knowledge proofs.

Optionally, the system may further comprise a non-structured data processing module for converting unstructured data sources into a structured text format, wherein said unstructured data is selected from a group comprising molecular profiles, imaging findings, Optical Character Recognition OCR-readable printed documents, and handwritten notes which are converted using a standardized ontology for further processing by the at least two agents.

Optionally, the system may further comprise an interface module for generating an interface for displaying and modifying patient profile parameters of a patient profile extracted by the at least two agents wherein the interface module is configured to display each of the outputs of the agents and the patient profile in a separate window with an addressable linkage to the underlying patient profile from which extraction was performed.

The interface module may be configured to display for a selected patient profile a probability score indicative of relative match evaluated by the consensus module for a plurality of potential clinical trials and conditions of the clinical trial criteria extracted by the evaluation agents, and an addressable linkage to the clinical trial information from which the extraction was performed.

In accordance with a second aspect of the present disclosure, there is provided a computer implemented method for matching a subject with one or more suitable clinical trials from a plurality of clinical trials.

The method comprises (i) receiving by one or more processors of a computer system and via a communication interface a patient profile of the subject comprising a plurality of different parameters and a plurality of clinical trials comprising clinical trial criteria with a plurality of different conditions; (ii) using an evaluation framework module to evaluate suitability of a patient profile for one or more of the plurality of clinical trials, wherein the evaluation framework comprises at least two evaluation agents executing on the one or more processors; wherein each evaluation agent is configured to have a specific analysis domain different from the other evaluation agent, wherein each evaluation agent is configured to use a large language model and wherein each evaluation agent is configured to: extract parameters from the patient profile and conditions of the clinical trial criteria according to the specific analysis domain of that agent and calculate a score indicative of the degree of match therebetween; and generate a ranked list of potential suitable clinical trials according to the score indicative of the degree of match for the specific analysis domain for that evaluation agent and the patient profile; (iii) resolving by a consensus module executing on the one or more processors discrepancies between the ranked trial lists for said patient profile generated by the at least two evaluation agents to determine a refined list comprising one or more suitable clinical trials by: (a) using a weighted voting module if conflicting evaluations exist among the ranked lists of potential suitable clinical trials by prioritising trials with analysis domain results derived by more accurate evaluation agents where the accuracy of the evaluation agent for a specific domain is stored in the memory of the computer system to determine the refined list of suitable clinical trials for the subject; and (b) if the score indicative of the degree of match of potential suitable clinical trials differs by a predetermined similarity difference, using an arbiter module configured to resolve conflicting evaluations and stored in the memory of the computer system for generating the refined list of suitable clinical trials for the subject; (iv) outputting the refined list of clinical trials for the patent profile via a user interface or device and receiving data indicative of the suitability of the subject to a specific clinical trial.

Optionally, the method may further comprises dynamically adjusting matching metrics of the at least two evaluation agents and the arbiter module based on the feedback data by adjusting weights assigned to specific patient parameters and logical parsing rules specific to each agent upon receiving data indicative of the suitability of the patient to a specific clinical trial.

Optionally, re-ranking by the arbiter module is performed by determining a custom probability score indicating suitability of at least some of the plurality of clinical trials for that subject.

Optionally, the potentially suitable clinical trials are filtered by predetermined mandatory exclusion criteria to exclude unsuitable clinical trials before generating the ranked list of the potential suitable clinical trials, wherein the predetermined mandatory exclusion criteria are selected from the group comprising molecular biomarkers, primary diagnosis, gender and age.

Optionally, each evaluation agent is configured to cite supporting text for criteria used in the determination of the evaluation score and in the absence of supporting text, the evaluation agent is configured to exclude that data from the evaluation score until external verification.

Optionally, the specific analysis domains of the evaluation agents are selected from a group comprising a molecular analysis domain; a disease evaluation domain; a demographic domain and a laboratory test results domain.

Optionally, the arbiter module comprises a first arbiter module configured for resolving conflicting evaluations for specific contextual patient parameters and a second arbiter module comprises a large language model configured for resolving conflicting evaluations for specific non-contextual patient parameters.

Optionally, in the molecular analysis domain the evaluation framework module may be configured for comparison of molecular patient parameters and molecular clinical trial conditions and to provide an indicative score representative of the degree of match therebetween together with supporting reasoning; in the disease evaluation domain the evaluation framework module may be configured for comparison of the disease stage patient parameters and the corresponding disease stage conditions of the clinical trials and to provide an indicative score representative of the degree of match therebetween together with supporting reasoning; and in the demographic domain the evaluation framework module may be configured for comparison of patient demographic parameters and the corresponding demographic parameters in conditions of the clinical trial; and to provide an indicative score representative of the degree of match therebetween together with supporting reasoning; and in the laboratory test domain the evaluation framework module may be configured for comparison of the patient laboratory results with corresponding laboratory tests conditions of the clinical trial; and to provide an indicative score representative of the degree of match therebetween together with supporting reasoning.

Optionally, 0 is assigned as the score if the subject is evaluated as ineligible for the clinical trial and 1 is assigned as the score if the patient profile parameters and the conditions of the clinical trial criteria match perfectly or assigning a number between these values according to the degree of evaluated match.

Optionally, the large language model types are selected from the group comprising Reasoning large language models, Multimodal models, Traditional large language models, and Agentic large language models.

Optionally, the data indicating the suitability of a patient profile to a corresponding clinical trial is selected from a group comprising real-world patient outcomes, expert annotations from clinicians, or simulated historical trial data.

Optionally, the method may further comprise removing personally identifiable information from patient profiles by zero-knowledge proofs.

Optionally, the method may further comprise converting unstructured data sources into a structured text format, wherein said unstructured data is selected from a group comprising molecular profiles, imaging findings, Optical Character Recognition readable printed documents, and handwritten notes which are converted using a standardized ontology for further processing by the at least two agents.

Optionally, the method may further comprise via a user interface displaying and modifying patient profile parameters of a patient profile extracted by the at least two agents wherein the user interface is configured to display each of the outputs of the agents and the patient profile in a separate window with an addressable linkage to the underlying patient profile from which extraction was performed.

Optionally, the interface may be configured to display for a selected patient record a probability score indicative of relative match evaluated for a plurality of potential clinical trials, and including conditions of the clinical trial criteria extracted by the evaluation module and an addressable linkage to the clinical trial information from which the extraction was performed.

In accordance with a third aspect of the present disclosure, there is provided a non-transitory machine-readable medium for matching a subject with one or more suitable clinical trials of a plurality of clinical trials, the non-transitory machine-readable medium storing instructions which, when executed by one or more processors of a computing system, causes the one or more processors to perform operations comprising the steps of the method as described above.

### BRIEF DESCRIPTION OF THE DRAWINGS

In order to describe the manner in which the above-recited and other advantages and features of the disclosure can be obtained, a more particular description of the principles briefly described above will be rendered by reference to specific embodiments thereof which are illustrated in the appended Figures. Understanding that these Figures depict only exemplary embodiments of the disclosure and are not therefore to be considered to be limiting of its scope, the principles herein are described and explained with additional specificity and detail through the use of the accompanying Figures.

Preferred embodiments of the present disclosure will be explained in further detail below by way of examples and with reference to the accompanying Figures, in which: -
**FIG.** 1 depicts an exemplary system for matching subjects for clinical trials according to an embodiment of the present disclosure.
**FIG. 2a** depicts a contextual representation of the exemplary schematic system of **FIG. 1****.**
**FIG. 2b** depicts an exemplary representation of various data types which may be include within a patient profile
**FIG. 2c** depicts performance of an exemplary Grok 3 in GPQA and MMMU benchmarks according to an embodiment of the present disclosure.
**FIG. 2d** depicts an example of a summary of molecular analysis domain of patient profiles
**FIG. 2e** depicts a set of relevant clinical trials identified for a patient according to one example of the present disclosure.
**FIG. 2f** depicts an example of re-ranking suitable clinical trials for the patient to provide patient-trail match decision according to one embodiment of the present disclosure.
**FIG. 3** depicts an exemplary simplified flow chart of the method for matching subjects for clinical trials according to an embodiment of the present disclosure.
**FIG. 4** depicts an exemplary user interface for an embodiment of the system of the present disclosure for displaying a comprehensive summary of medical features according to an embodiment of the present disclosure.
**FIG. 5** depicts an exemplary user interface for an embodiment of the system of the present disclosure for displaying matching decision results for a specific patient according to an embodiment of the present disclosure.
**FIG. 6a to FIG. 7b** depict examples of structured patient summary of two exemplary scenarios and exemplary identified matching results.

### DETAILED DESCRIPTION OF THE PREFERRED EMBODIMENTS

Various embodiments of the disclosure are discussed in detail below. While specific implementations are discussed, it should be understood that this is done for illustration purposes only. A person skilled in the relevant art will recognize that other components and configurations may be used without departing from the spirit and scope of the disclosure.

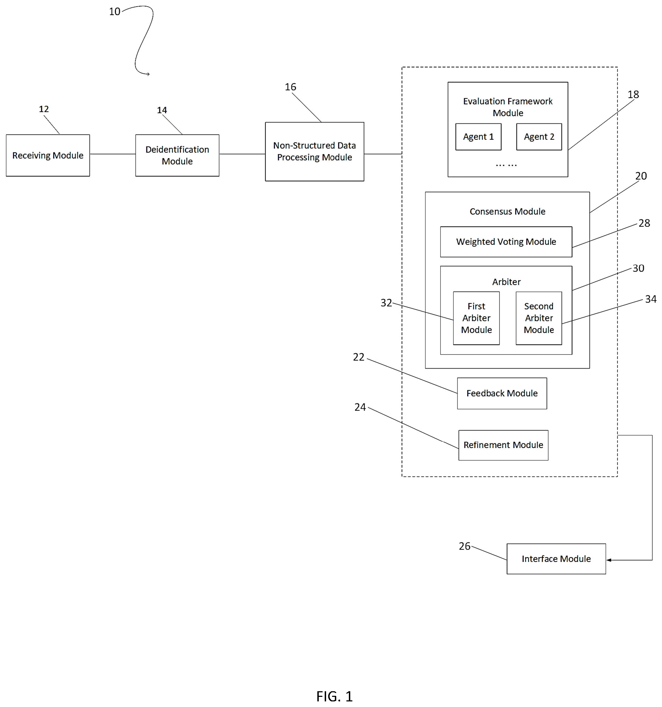

Referring to **FIG. 1****,** there is shown a schematic representation of a system **10** for matching suitable subjects for clinical trials.

In one embodiment, the system **10** is configured to identify one or more appropriate subjects for one or more clinical trials from a plurality of patient profiles. In some embodiments, the patient profiles may be de-identified. The system **10** comprises a receiving module **12,** an evaluation framework module **18,** a consensus module **20,** a feedback module **22** and a refinement module **24** (optional). The receiving module **12** may be configured to receive patient profiles comprising a plurality of different parameters and a plurality of clinical trials comprising clinical trial criteria with a plurality of different conditions.

Various parameter domains of patient profiles are analysed to determine whether a patient qualifies for a clinical trial. The evaluation framework module **18** may comprises at least two agents, for example Agent 1, Agent 2, Agent n....... for different specific analysis domains. Adopting multiple agents can provide a structured and comprehensive approach to assess patient eligibility for clinical trials based on multiple analysis domains of patient profile.

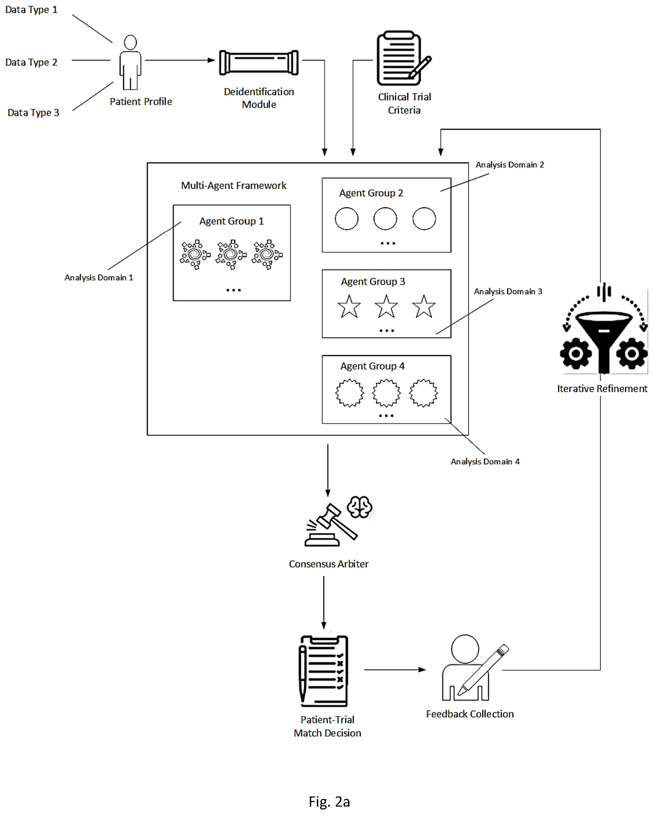

In an embodiment as depicted in **FIG. 2a****,** the evaluation framework module **18** includes four specialized agents, i.e., Agent 1, Agent 2, Agent 3 and Agent 4 with each tasked with evaluating a specific analysis domain of patient profile, i.e., Analysis Domain 1, Analysis Domain 2, Analysis Domain 3, Analysis Domain 4.

In Analysis Domain 1, which may be example the molecular analysis domain, Agent 1 of the evaluation framework module **18** is configured for comparison of molecular patient parameters and molecular clinical trial conditions and to provide an indicative score representative of the degree of match therebetween together with supporting reasoning.

In Analysis Domain 2, which may be for example a disease evaluation domain, Agent 2 of the evaluation framework module **18** is configured for comparison of the disease stage patient parameters and the corresponding disease stage conditions of the clinical trials and to provide an indicative score representative of the degree of match therebetween together with supporting reasoning.

In Analysis Domain 3, for example the demographic domain, Agent 3 of the evaluation framework module **18** is configured for comparison of patient demographic parameters and the corresponding demographic parameters in conditions of the clinical trial; and to provide an indicative score representative of the degree of match therebetween together with supporting reasoning.

In Analysis Domain 4, for example the laboratory test domain, Agent 4 of the evaluation framework module **18** is configured for comparison of the patient laboratory results with corresponding laboratory tests conditions of the clinical trial; and to provide an indicative score representative of the degree of match therebetween together with supporting reasoning.

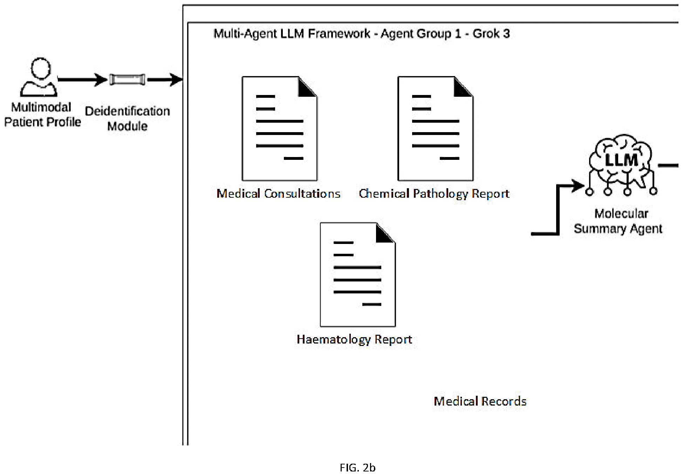

**FIG. 2b** shows an exemplary representation of various data types which may be include within a patient profile. In this example, the date of patient profile is obtained from a subset of scanned medical records of a patient, including consultation notes with three documents outlining the patient's medical consultations; chemical pathology report with details of the patient's chemical and metabolic health and haematology report focusing on blood-related health metrics. It is noted that the specifics of the representations depicted is not important, and these information may be in a combination of structured or unstructured data formats. Additional and or alternative patient data may also be provided without limitation.

Agents are utilised to evaluate the data of the patient profile in each of the particular analysis domains. Each agent may be assigned to certain features of an analysis domain and may employ independent large language model LLM specialised for different specific analysis domain so as to simplify and enhance the analysis. For the agents within the evaluation framework module 18, the type of the large language model utilized for specified analysis domains can either be of the same type or different to each other, contingent upon the performance of the large language model for that analysis domain.

In one embodiment as depicted in **FIG. 2a** and discussed above, the large language model of Agent 1, Agent 2, Agent 3 and Agent 4 specialised for different analysis domains may be selected from the group comprising Reasoning large language models, Multimodal models, Traditional large language models, and Agentic large language models.

As known to a person skilled in the art, reasoning large language models are optimized for logical reasoning, problem-solving, and complex tasks like mathematics, coding, and scientific analysis. Reasoning large language models often excel at step-by-step thinking and tackling structured problems. For example, OpenAl o1, DeepSeek R1, Grok, Google's Gemini, Meta's Llama 3.1, Anthropic's Claude 3.5 Sonnet, Mistral's Pixtral Large are commonly known Reasoning large language models.

Multimodal Models are generally adopted to process and generate multiple types of data, such as text, images, audio, or video. Multimodal Models can provide an idea performance for tasks requiring integration of diverse inputs, like image captioning or cross-modal generation. Gemma 3, GPT-4 Omni (GPT-4o), Grok-3, CLIP, Flamingo, BLIP-2 are commonly known Multimodal Models, but it would be appreciated that other multimodal models could be used without departing from the scope of the present disclosure.

Traditional large language models are general-purpose models designed for text generation, understanding, and a wide range of natural language processing (NLP) tasks. They are typically trained on massive da-tasets of text and excel at tasks like conversation, translation, and content creation. ChatGPT (GPT-3.5, GPT-4), Llama (Llama 2, Llama 3), BERT, T5, Mistral 7B are commonly known traditional large language models.

Agentic large language models are an emerging category of large language models which can perform actions, use tools (e.g., web search, code execution), and interact with external systems. Agentic large language models are more autonomous and suited for dynamic, task-oriented workflows. OpenAl o3, OpenAl o4-mini, Mistral's models with function calling (e.g., Mistral Small 3), An-thropic's Claude are commonly known Agentic large language models.

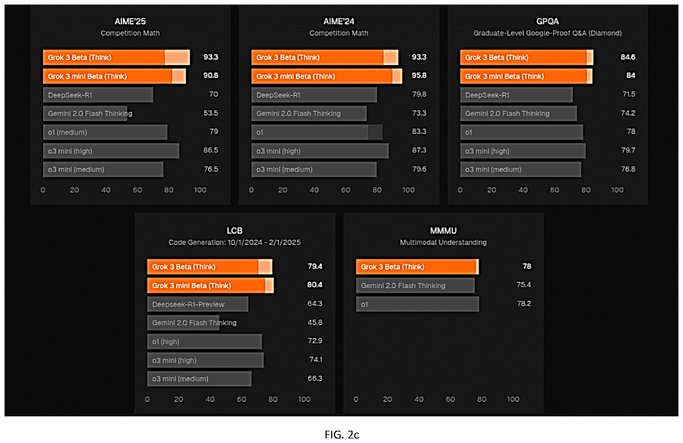

In this example, the Grok 3 based large language model is utilized to evaluate the patient's data from molecular aspect due to its superior performance. Grok 3's top performance in GPQA and MMMU benchmarks, as shown in **FIG. 2c****,** makes it a good choice for parsing patient medical records and generating molecular summaries. Grok 3's high GPQA score reflects advanced reasoning capabilities, ideal for synthesizing complex information and accurately identifying specific genetic mutations. Meanwhile, its leading MMMU score ensures proficiency in handling multimodal data-like text, tables, and images found in medical records-enabling seamless interpretation of diverse formats.

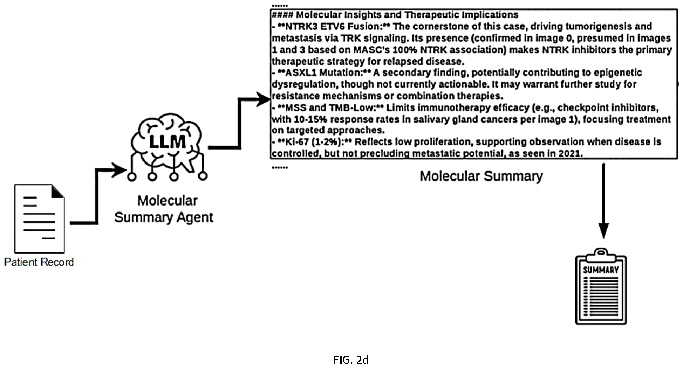

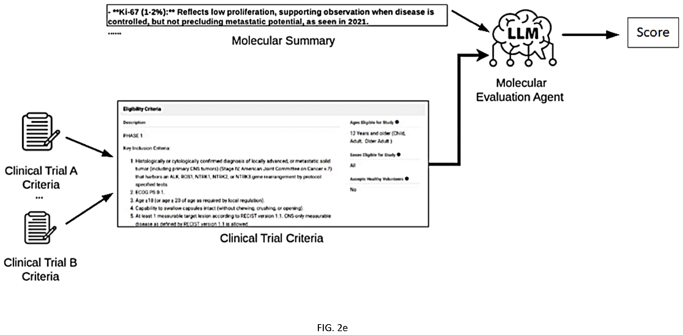

**FIG. 2d** depicts an example of a summary of molecular analysis domain of patient profiles. The summary agent Grok 3 parses medical records and identifies molecular markers, such as specific genetic mutations. After extracting this information, the summary agent updates the patient's summary in its designated molecular area, as shown in **FIG. 2e****.** If no summary exists yet (e.g., for a new patient), the agent creates a summary using the extracted data. These summaries act as concise, current snapshots of the patient's status in each category.

An example system prompt for the Molecular Evaluation Agent may be structured as follows:
[System Prompt]: "You are a clinical trial eligibility evaluator specializing in molecular criteria. Given a patient's molecular summary and a clinical trial's molecular eligibility criteria, assess whether the patient satisfies each molecular criterion. For each criterion, provide: (1) a match score between 0 and 1 where 0 indicates definite ineligibility and 1 indicates a perfect match; (2) a confidence value of -1 (unreliable source data), 0 (uncertain), or 1 (reliable); and (3) a reasoning statement citing the specific patient data supporting your assessment. Output as JSON: {criteria_assessments: [{criterion: string, score: float, confidence: int, reasoning: string, supporting_evidence: string}]}."
[Patient Molecular Summary]: The structured molecular summary generated by the Molecular Summary Agent is injected here.
[Trial Criteria]: The molecular eligibility criteria for the specific clinical trial are injected here.
[Instruction]: "Evaluate the patient against each molecular criterion listed above. Base your assessment only on the provided patient data. If data is missing for a criterion, assign a score of 0 and confidence of -1."

Similar prompt templates may be constructed for the Disease Evaluation Agent, the Basic Demographic Evaluation Agent, and the Lab Test Evaluation Agent, each adapted to their respective analysis domains with domain-specific extraction schemas and evaluation criteria.

The flexibility of the evaluation framework module **18** of an embodiment of the present disclosure is a key strength, allowing the system and method described herein to incorporate additional LLM groups or agents as needed. The performance of different available LLMs on different benchmarking datasets and the best performing LLM family can be selected to analyze patient's data from different analytical perspectives. For example, OpenAl Chat-GPT-40 may be selected as the best model for analyzing patient lab test records, Lab Test Summary Agent from OpenAl Chat-GPT-40 LLM group would be selected to evaluate the patient's data from lab test aspect and generate lab test summary. Potential alternatives could include models like DeepSeek R1, OpenAl Chat-GPT-4o, OpenAl o1, the Llama family from Meta, or the Gemma family from Google. This scalability ensures the system can adapt to new language models and newly developed research.

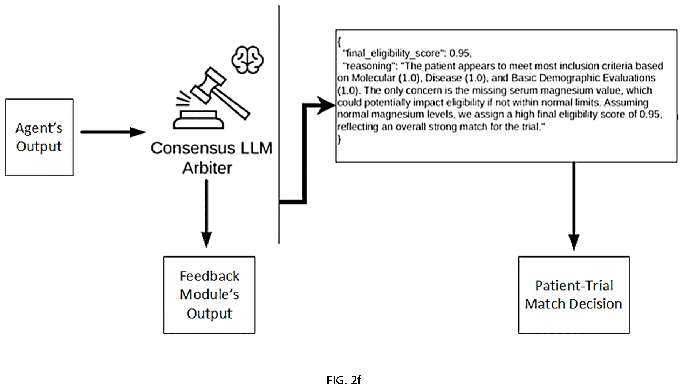

Once a set of relevant clinical trials, for example as shown in **FIG. 2f****,** is identified for a patient, the evaluation framework module **18** assesses eligibility by leveraging the patient summaries and trial-specific criteria.

The potential suitable clinical trials may be filtered by predetermined mandatory criteria to exclude unsuitable clinical trials before generating the ranked list of the potential suitable clinical trials. For example, the predetermined mandatory criteria may include molecular biomarkers, primary diagnosis, gender and age.

The eligibility criteria for each trial are retrieved and distributed to the appropriate evaluation agents. Each evaluation agent evaluates the patient against the criteria relevant to its domain, using the corresponding patient.

For example, the Molecular Evaluation Agent may be configured to compare the patient's molecular summary to the trial's molecular requirements (e.g., presence of a specific mutation). Advantageously it may be configured to assign an eligibility score between 0 and 1. A score of 0 indicates the patient is definitely ineligible based on molecular factors and a score of 1 indicates from a molecular perspective the patient would be a good potential subject for the trial. Alongside the score, the evaluation agent may be configured to provide a reasoning statement explaining its assessment (e.g., "The patient lacks the required BRCA1 mutation").

The Disease Evaluation Agent, Basic Demographic Evaluation Agent, and Lab Test Evaluation Agent follow the same process, each focusing on their respective criteria (e.g., disease stage, age range, or lab thresholds) and generating their own scores and reasonings.

In this example, the system **10** generates a score of 0 if the patient is evaluated as ineligible for the clinical trial. Similarly a score of 1 may be generated if the patient parameters and the conditions of the clinical trial criteria are a strong match or a number between these values according to the degree of evaluated match.

The system **10** may further comprise a deidentification module **14** configured to remove personally identifiable information PII from patient profiles by zero-knowledge proofs for obtaining deidentified patient profiles. This allows the system **10** to determine if eligible patients exist without revealing individual patient details until explicit consent is obtained.

Zero-knowledge proofs, as a commonly used technical means in this field (see prior art documents such as US20180013562A1 and the paper "Health-zklDM: A Healthcare Identity System Based on Fabric Blockchain and Zero - Knowledge Proof" (available at https://www.mdpi.com/1424 - 8220/22/20/7716)), is a cryptographic technology that allows one party (the prover) to prove to another party (the verifier) that a certain statement is true without revealing any additional information other than the truth of the statement. In the present disclosure, using zero-knowledge proofs can ensure the privacy and security during the data interaction process.

To mitigate the risk of LLM hallucination in clinical trial matching assessments, the system 10 employs three complementary strategies.

First, citation grounding requires each agent to cite specific text chunks from the KGDB node supporting its assessment; any criterion assessment that lacks a valid source reference to the patient's clinical records is automatically flagged as unsupported and excluded from the scoring calculation until reviewed.

Second, cross-agent validation is performed where analysis domains overlap (e.g., disease stage may be referenced in both the molecular and disease evaluation domains); when two agents produce conflicting assessments for overlapping criteria, the discrepancy is flagged and escalated to the arbiter 30 for resolution.

Third, human-in-the-loop verification is triggered for mandatory criteria (specifically molecular biomarkers and primary diagnosis) when the agent's confidence value is 0 (uncertain) or -1 (unreliable); such assessments are flagged for clinician review before finalizing eligibility decisions, ensuring that high-stakes determinations are not made solely on uncertain LLM outputs.

In one embodiment, the evaluation framework module **18** is also configured to extract disparate parameters from said deidentified patient profiles and disparate conditions of the clinical trial criteria according to the specific analysis domain of that agent and evaluate a score indicative of the degree of match therebetween; and generate for at least some of said set of deidentified patient profiles, a ranked list of potential suitable clinical trials according to the evaluated match score for the specific analysis domain for that agent.

The consensus module **20** may be configured to resolve discrepancies between the ranked trial lists provided by agents within at least one of said set of deidentified patient profiles to determine a refined list comprising one or more suitable clinical trials.

The consensus module **20** may comprise a weighted voting module **28** and an arbiter 30. It may be configured to use the weighted voting module **28** if conflicting evaluations exist among the ranked lists of potential suitable clinical trials by prioritising trials with analysis domain results derived by more accurate agents to determine the refined list of suitable clinical trials for the patient. The custom probability score dynamically weights specific analysis domain of the at least two agents based on the trial criteria; For example, if trial A's criteria stress more about molecular aspects, the arbiter agent would dynamically weight the molecular agent's specific analysis domain more importantly.

If the evaluated match score of potential suitable clinical trials differs by a predetermined similarity difference, it may use the arbiter **30** which was trained to resolve conflicting evaluations to generate the refined list of suitable clinical trials for the patient.

The evaluation framework module **18** may be trained using the results of expert human interviews involving research nurses to provide feedback and help identify which analysis domains of a patient profile are most critical when evaluating eligibility for clinical trials.

In an exemplary embodiment during the implementation of system **10,** based on the results of expert interviews, molecular Information in the molecular analysis domain may be determined as the most significant feature. This may be most significant as molecular data, including genetic mutations and biomarkers, typically has a direct and intrinsic correlation with the specific research focus of oncology trials. Disease related Information in the disease evaluation domain, which encompasses elements such as diagnosis, disease stage, and progression, is identified as a highly significant factor, yet it is of slightly lower importance compared to Molecular Information.

As for basic Demographics in the demographic domain, such as age, gender, and ethnicity are identified as relevant factors, but having relatively less impact on the determination of eligibility. Regarding lab test results in the laboratory test domain, lab test results are regarded as the least critical category as they are generally liable to vary over time.

The identified factors of analysis domains and the respective levels of significance may be updated, developed, modified and expanded time to time according to the developments in the field.

It should be understood that persons skilled in the art could use other analysis domains of patient profile with other levels of significance/importance, not departing from the scope of the present disclosure.

The feedback module **22** may be configured to collect external feedback data indicative of the suitability of a subject to a specific clinical trial. For example, the external feedback data indicating the suitability of a patient profile to a corresponding clinical trial is selected from a group comprising real-world patient outcomes, expert annotations from clinicians, or simulated historical trial data.

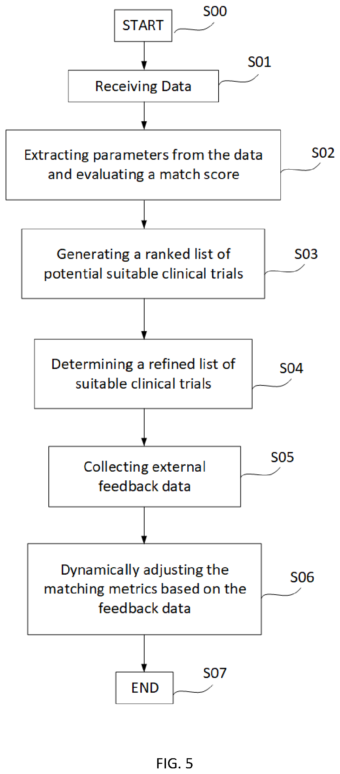

In one embodiment, the system **10** may further comprise the refinement module **24** configured to dynamically adjust the matching metrics of the at least two agents and the arbiter based on the feedback data by adjusting weights assigned to specific patient parameters and logical parsing rules specific to each agent upon multiple iterations of the step **S02** to **S06** of the method as depicted below by referring **FIG. 5****.**

The arbiter module **30** is used to re-rank suitable clinical trials for the patient to provide patient-trail match decision as shown in **FIG. 2f****.**

In one embodiment, the re-ranking is performed by determining a custom probability score indicating suitability of at least some of the plurality of clinical trials for that patient. The arbiter module **30** may comprise a first arbiter module **32** for resolving conflicting evaluations for specific contextual patient parameters and a second arbiter module **34** having a large language model for resolving conflicting evaluations for specific non-contextual patient parameters.

In the clinical field, specific contextual patient parameters may refer to various factors and data related to patients/subjects that are relevant in a particular clinical context. They can include the following aspects:

Demographic parameters: Such as age, gender, ethnicity, and occupation. These factors can provide basic information about the patient and may be related to the incidence and prognosis of certain diseases.

Medical history parameters: Including past medical conditions, surgeries, allergies, and family medical history. Understanding a patient's medical history is crucial for diagnosing current conditions and formulating treatment plans.

Clinical symptoms and signs parameters: These are the direct manifestations of the patient's condition, such as pain, fever, cough, shortness of breath, and abnormal physical examination findings. These parameters help doctors identify the possible causes of the disease.

Laboratory test parameters: Results of blood tests, urine tests, imaging tests (such as X - rays, CT scans, MRls), and pathological examinations. These tests can provide objective data on the patient's physical condition and help in the diagnosis and monitoring of diseases.

Treatment - related parameters: Such as the type of treatment received (e.g., medication, surgery, radiotherapy), dosage, treatment duration, and treatment response. These parameters are important for evaluating the effectiveness of treatment and adjusting the treatment plan.

Psychosocial parameters: Including the patient's psychological state (e.g., anxiety, depression), social support system, and lifestyle factors (such as diet, exercise, smoking, and alcohol consumption). These factors can affect the patient's recovery and quality of life and suitability for the trial.

In the clinical field, non-contextual patient parameters may refer to factors that are not directly related to a specific clinical context or the patient's current condition. Some examples include:

General population statistics: Parameters such as average life expectancy, disease incidence rates in the general population, and prevalence of certain genetic traits in a large - scale population. These are broad - based data that do not pertain to an individual patient's specific circumstances.

Standardized medical norms and guidelines: These are established criteria and recommendations for diagnosing and treating diseases, such as normal ranges for laboratory tests, recommended treatment protocols for various conditions, and staging systems for cancers. While they provide a framework for clinical practice, they are not specific to an individual patient's context.

Historical medical data from other patients: Aggregated data from previous patients who may have had similar diseases or treatments, but without direct relevance to the current patient's specific situation. This could include data from clinical trials, epidemiological studies, or hospital databases that are used for research and benchmarking purposes rather than for immediate patient care.

Generic medical knowledge and theories: Basic medical sciences knowledge, such as the structure and function of the human body, pathophysiological mechanisms of diseases, and principles of pharmacology. Although essential for understanding and treating diseases, this knowledge is not tailored to the specific context of an individual patient.

External environmental factors not directly related to the patient: Such as air quality, pollution levels, and seasonal variations in disease occurrence, which may have an impact on population - wide health trends but do not directly affect an individual patient's condition in a specific clinical context.

The system may further comprise a non-structured data processing module **16** for converting unstructured data sources into a structured text format, wherein said unstructured data is selected from a group comprising molecular profiles, imaging findings, Optical Character Recognition OCR-readable printed documents, and handwritten notes which are converted using a standardized ontology for further processing by the at least two agents.

In contrast to conventional methodologies such as Named Entity Recognition (NER) and rule-based data extraction, which are frequently cited in prior art documents, the proposed solution of the present disclosure uses practical, real-world experiences rather than theoretical constructs alone.

Traditional NER techniques are designed within the field of natural language processing to identify and categorize specific named entities within unstructured text. Similarly, rule-based data extraction relies on a set of predefined rules crafted to isolate and retrieve particular pieces of information from a dataset.

Despite their widespread use, these traditional approaches exhibit notable limitations that can compromise their effectiveness in processing complex or nuanced data. For instance, these approaches often fail to accurately identify shorthand terms or abbreviations that are prevalent in specialized fields, such as medical. Additionally, these methods can fail to discern subtle contextual differences within the data, such as distinguishing between a treatment that is merely planned or suggested versus one that a patient is actively undergoing according to doctor notes. Such misinterpretations can lead to errors in data analysis, reducing the reliability and utility of the extracted information.

To overcome these deficiencies, the approach of the present disclosure uses Multimodal Large Language Models, with models like Gemma3 serving as a representative and non limiting example. These models are engineered to process and interpret a wide variety of unstructured data types within a single, cohesive framework.

Unlike traditional methods that may require separate tools or processes for different data formats, Multimodal Large Language Models are capable of uniformly handling diverse inputs, including printed texts that are readily legible through Optical Character Recognition (OCR), handwritten notes that pose challenges to OCR due to variability in script, and even images that traditional approaches might find difficult to analyze without specialized preprocessing.

The data extraction process may include ingestion by the Multimodal Large Language Model of the unstructured data, analyzing this data comprehensively regardless of its original format-be it text, handwriting, or visual imagery.

This initial processing phase ensures that all data types are treated with equal consideration, eliminating the need for format-specific adjustments. Following this, the evaluation framework module **18** generates a detailed summary tailored to a specific analysis domain, such as molecular aspect.

This summary is not a mere condensation of the input data; rather, the model refines and clarifies the information, presenting the information in a way that enhances its accuracy and relevance to the chosen domain. Subsequently, this detailed summary serves as the foundation for extracting structured text features, using a standardized ontology for example. Finally, the detailed summary and these structured text features are transmitted to agents for further processing.

The inventiveness of the approach of the present disclosure can be appreciated by several distinctive and advantageous features that set it apart from traditional methodologies.

First, the use of Multimodal Large Language Models of the evaluation framework module 18 enables the uniform treatment of all unstructured data types, irrespective of their source or complexity. In conventional systems, processing printed text might rely on OCR, while handwritten text or images could necessitate entirely separate tools or manual intervention, complicating the workflow and introducing potential points of failure. The proposed solution, by contrast, leverages trained multimodal models to seamlessly process all such inputs-whether easily OCR-readable printed documents, challenging handwritten notes, or intricate images-within a single, integrated model. This uniformity not only simplifies data handling but also broadens the applicability of the solution across diverse datasets.

Second, defining Patient, Condition, Treatment, and Encounter as nodes in a knowledge graph database (KGDB) offers significant advantages over a vector-only Retrieval-Augmented Generation (RAG) system that is commonly used in the LLM field. The proposed KGDB schema as depicted in the algorithm details set out below enables precise semantic queries, such as identifying patients with specific conditions and treatments. Vector-only RAG relies on text embedding similarity, often missing relational context, and require the LLM to infer relationships, risking hallucinations. The proposed KGDB implementation supports incremental updates such as new clinical encounters or treatments, unlike RAG's costly re-indexing whenever a new text is added.

Third, the methodology described herein incorporates an iterative refinement process that further distinguishes it from static, one-pass traditional approaches. Through the feedback loop, the models learn from historical and prospective trial enrolment records, adapting to the data over time. This continuous improvement mechanism ensures that the solution becomes increasingly accurate and reliable with each iteration, offering a dynamic adaptability that traditional methods lack.

Optionally, the system **10** may further comprise an interface module **26** for generating an interface for displaying and modifying parameters of a deidentified patient record extracted by the at least two agents. The interface module may be configured to display each of the outputs of the agents analysing a deidentified patient record in a separate window with an addressable linkage to the underlying patient record from which extraction was performed. Optionally, the interface module may be configured to display for a selected deidentified patient record a probability score indicative of relative match evaluated by the consensus module for a plurality of potential clinical trials, and including conditions of the clinical trial criteria extracted by the at least two agents and an addressable linkage to the clinical trial information from which the extraction was performed.

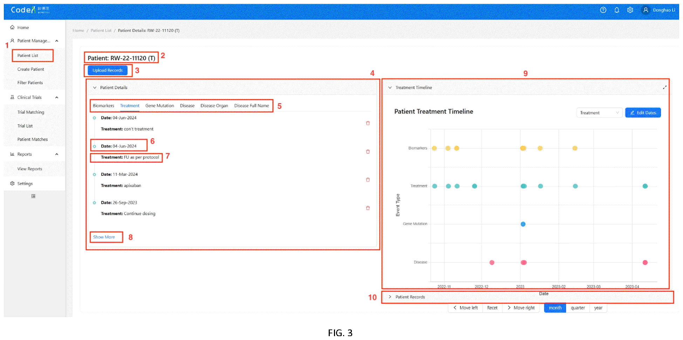

**FIG. 3** depicts an exemplary user interface for an embodiment of the system of the present disclosure for displaying a comprehensive summary of medical features according to an embodiment of the present disclosure. In particular, **FIG. 3** presents a graphical user interface (GUI) configured to display a comprehensive summary of medical features derived from an input patient profile following deidentification and analysis by specialized LLM agents. The patient profile integrates multimodal data, including text-based records (e.g., consultation notes, lab reports) and non-text data (e.g., molecular profiles, imaging data), which are converted into a text format suitable for LLM processing. The GUI is structured into multiple functional sections, each serving a distinct purpose in presenting and managing patient data, as detailed below.

In **FIG 3****,** element #1, denotes a vertical sidebar. This sidebar enables users to navigate between different views and outputs generated by the system at various processing stages. The sidebar includes selectable options such as "Patient List," "Create Patient," "Filter Patients," "Clinical Trials," "Trial Matching," "Trial List," "Patient Matches," and "View Reports." Each option corresponds to a specific operational mode, allowing users to access distinct functionalities of the system. For example, selecting the option associated with element #1 transitions the interface to a detailed view of a patient profile, wherein medical features processed and analyzed by independent LLM agents across specialized domains are displayed, as described in the section System Architecture.

Adjacent to the sidebar, a second portion, labeled as element #2, displays a deidentified patient identifier, such as "Patient: RW-22-1120 (T)." This unique reference code is utilized for internal system tracking, ensuring compliance with privacy standards by excluding personally identifiable information. The deidentified identifier is critical for maintaining patient confidentiality while enabling the system to reference and manage individual profiles effectively.

Positioned near the patient identifier, element #3 consists of an "Upload Records" button. This interactive component allows users to submit new medical records to the associated patient profile. Upon activation, the uploaded records-encompassing multimodal data such as text (e.g., consultation notes) and non-text data (e.g., molecular profiles, imaging)-are processed and analyzed by specialized LLM agents, as outlined in the section System Architecture. These agents extract relevant medical features, and the resulting data is integrated into the patient profile, updating the display within GUI to reflect the most current information.

Element #4 is dedicated to the detailed presentation of the patient profile. This section showcases medical features extracted from the uploaded records by the LLM agents, providing a comprehensive overview of the patient's medical history and clinical status. The content within this section is dynamically updated based on user interactions and selections made via a tabbed interface, labelled as element #5.

Element #5 comprises a series of selectable tabs that enable users to view distinct aspects of the patient profile, including "Biomarkers," "Treatment," "Gene Mutation," "Disease," "Disease Organ," and "Disease Full Name." Each tab corresponds to a specialized analysis performed by independent LLM agents focusing on different medical domains as outlined in the section System Architecture. For instance, molecular agents process and analyze data related to biomarkers and gene mutations, outputting results to the respective tabs, while disease agents focus on disease states, treatments, and affected organs, populating the corresponding tabs with their findings. This tabbed structure enhances accessibility, allowing users to efficiently explore specific facets of the patient profile as processed by the multi-agent LLM framework.

When the "Treatment" tab within element #5 is selected, the section labeled as element #4 displays a chronologically sorted list of treatments administered to the patient, as extracted by disease-focused LLM agents from the uploaded medical records. Each entry in this list comprises two components: a date-time stamp, as indicated by element #6 (e.g., "Date: 04-Jun-2024"), and the corresponding treatment name, as indicated by element #7 (e.g., "Treatment: FU as per protocol"). Given the potential volume of treatment data-particularly in cases where patient records span hundreds of pages-a "Show More" button, labeled as element #8, is provided. This button enables the progressive disclosure of additional treatment entries, ensuring that the interface remains uncluttered while offering comprehensive access to the patient's treatment history.

A separate visualization area, labeled as element #9, offers an alternative representation of the patient data in the form of a "Patient Timeline." This timeline graph plots medical events-such as treatments, biomarkers, gene mutations, and disease states-as colored dots along a temporal axis. Each event type is distinguished by a unique color (e.g., teal for "Treatment," yellow for "Biomarkers," blue for "Gene Mutation," and pink for "Disease"). The x-axis of the timeline is adjustable via navigation controls, including "Move left," "Reset," "Move right," and time granularity options ("month," "quarter," "year"), allowing users to zoom in on specific periods or pan across the data range, which in this example extends from 2022-11 to 2023-04. This timeline provides a visual summary of the patient's medical history, facilitating the identification of temporal patterns or significant events.

Finally, element #10, labeled "Patient Records," is a tab or section that, when selected, displays the original source medical records from which specific medical features were extracted by the LLM agents. For exampleRefer, if a user clicks on a treatment name within the list (e.g., element #7, such as "apixaban"), selecting the "Patient Records" tab reveals the corresponding consultation note or other record where that treatment was documented. This feature enhances transparency and traceability, enabling users to verify the source of the extracted data and conduct further analysis as needed.

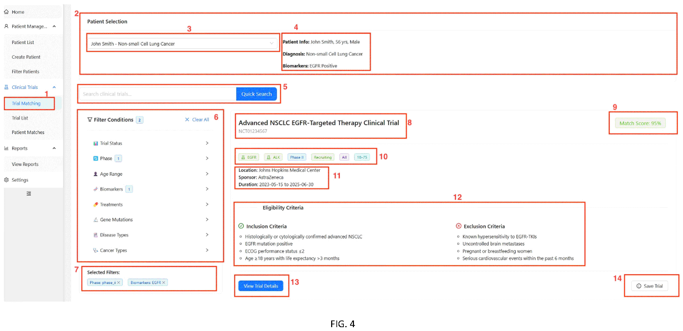

**FIG. 4** depicts an exemplary user interface for an embodiment of the system of the present disclosure for displaying matching decision results for a specific patient according to an embodiment of the present disclosure.

In particular, **FIG.4** depicts a further graphical user interface (GUI) configured to display Patient-Trial Match Decision results for a specific patient, generated through processing and analysis by distinct groups of LLM agents (e.g., Gemma v3, DeepSeek R1) within the disclosed multi-agent LLM framework. The trial matching results presented in GUI of **FIG.4** are the outcome of post-processing by a consensus module, as detailed herein.

A first portion of GUI in **FIG. 4****,** located on the left side of the interface and designated as element #1, comprises a vertical sidebar containing navigational options. Within this sidebar, the "Trial Matching" tab, positioned under the "Clinical Trials" group, serves as the entry point to the Patient-Trial Match Decision UI page. Selecting this tab navigates the user to the interface depicted in **FIG. 4****,** enabling access to trial matching results tailored to a selected patient.

Element #2 is dedicated to display information about the currently selected patient whose trial matching results are being evaluated. For authorized and authenticated users, non-deidentified patient information is presented in this section, ensuring that healthcare professionals can access comprehensive patient details while adhering to privacy restrictions for unauthorized users.

Element #3, configured as a dropdown menu, enables the selection of a specific patient from a list. In the illustrative example, a fictional patient named "John Smith" is selected, with "Non-small Cell Lung Cancer" identified as the primary diagnosis from the patient's profile. Element #4 presents essential patient information that are summarised by the LLM agents.

Element #5 consists of a search bar that facilitates custom searches for clinical trials relevant to the selected patient. This feature allows users, such as doctors, to leverage their expert knowledge-beyond the data automatically extracted by the system from uploaded patient records-to identify additional trials that may be suitable for the patient. The search bar enhances the system's flexibility by supporting manual trial exploration.

Element #6 provides a condition filter interface, where medical features extracted by LLM agents from the patient's medical records are automatically applied as filter criteria. These features include disease phases, age range, biomarkers, treatments, gene mutations, disease types, and cancer types, as well as trial-specific parameters such as trial status (e.g., "available," "active but not recruiting"). The filter options are accessible via a dropdown menu, enabling users to refine the trial matching results based on specific clinical or trial attributes. Element #7 displays the currently selected filter conditions (e.g., "Phase II," "Biomarkers: EGFR Positive"), each of which can be individually removed by selecting an "x" icon adjacent to the condition. Additionally, a "Clear All" option, located at the top right of element #6, allows users to reset all applied filters simultaneously, restoring the unfiltered state of the trial results.

The right side of GUI in **FIG.4** is dedicated to presenting the trial matching results, which are generated through the collaborative analysis of distinct LLM agent groups and refined by the consensus module, as described in the System Architecture section. Each matching trial is displayed in a structured format, beginning with element #8, which presents the main title of the trial (e.g., "Advanced NSCLC EGFR-Targeted Therapy Clinical Trial") and its unique identifier (e.g., "NCT01234567"). Element #9 displays a custom probability score, calculated by the consensus module, indicating the trial's suitability for the selected patient. This score is visually emphasized, for instance, as "Match Score: 95%" within a highlighted badge, offering users an immediate assessment of the trial's relevance.

Element #10 provides a summary of the trial's basic information, including related biomarkers (e.g., "EGFR and ALK mutations"), the cancer phase targeted by the trial (e.g., "Phase II"), the current trial status (e.g., "recruiting"), gender eligibility (e.g., "all genders"), and the age range of eligible participants (e.g., "18 to 75 years"). This concise overview enables users to quickly evaluate the trial's key attributes without requiring further navigation.

Additional administrative details of the trial are presented in element #11, including the trial location(s) (e.g., "Johns Hopkins Medical Center"), sponsor(s) (e.g., "AstraZeneca"), and duration (e.g., "2023-05-15 to 2025-03-30"). These details support logistical planning, such as patient referral or coordination with trial sites, enhancing the practical utility of the interface.

Element #12 lists the eligibility criteria for patient enrolment in the trial, which are critical inputs-alongside the patient's comprehensive profile-for calculating the probability score shown in element #9. These criteria may include inclusion requirements (e.g., "EGFR mutation positive," "age ≥18 years") and exclusion conditions (e.g., "uncontrolled brain metastases"), providing users with a clear understanding of the trial's enrolment parameters and the basis for its suitability assessment.

A "View Trial Details" button, designated as element #13, is provided for each trial, enabling users to access additional information not displayed in the initial summary. This may include detailed trial protocols, objectives, or other relevant data points, offering a deeper exploration of the trial's specifics as needed.

Element #14, the "Save Trial" button, allows users-such as healthcare professionals-to save a trial for future reference or referral. This functionality is particularly valuable when a user determines that a trial returned by the system may be a strong candidate for the patient, facilitating efficient tracking and follow-up actions.

Referring to **FIG. 5** there is depicted an exemplary simplified flow chart of the method for matching subjects for clinical trials according to an embodiment of the present disclosure.

As depicted, the method starts at step **S00.** At step **S01,** data is received.

The data includes deidentified patient profiles comprising a plurality of different parameters and a plurality of clinical trials comprising clinical trial criteria with a plurality of different conditions.

At step **S02,** parameters from the data are extracted and a match score is evaluated.

In the exemplary embodiment, disparate parameters of received deidentified patient profiles and disparate conditions of the clinical trial criteria according to the specific analysis domain of that agent are extracted from the data and a score indicative of the degree of match therebetween is evaluated

An evaluation framework module comprising at least two agents is used. Each agent employs independent large language model specialised for different specific analysis domains to extract disparate parameters from said deidentified patient profiles and disparate conditions of the clinical trial criteria according to the specific analysis domain of that agent.

The evaluation framework module evaluates a score indicative of the degree of match between deidentified patient profiles and the clinical trial criteria for at least some of said set of deidentified patient profiles.

At step **S03,** a ranked list of potential suitable clinical trials is generated.

The ranked list of potential suitable clinical trials is generated according to the evaluated match score for the specific analysis domain for that agent.

At step **S04,** a refined list of suitable clinical trials is determined. discrepancies between the ranked trial lists within at least one of said set of deidentified patient profiles are resolved so as to determine a refined list of suitable clinical trials.

If conflicting evaluations exist among the ranked lists of potential suitable clinical trials, a weighted voting module is used by prioritising trials with analysis domain results derived by more accurate agents to determine the refined list of suitable clinical trials for the patient.

If the evaluated match score of potential suitable clinical trials differs by a predetermined similarity difference, an arbiter trained to resolve conflicting evaluations is further used to generate the refined list of suitable clinical trials for the patient.

At Step **S05,** external feedback data is collected.

The external feedback data may indicate the suitability of a subject to a specific clinical trial.

At Step **S06,** the matching metrics are dynamically adjusted based on the feedback data According to the feedback data, the matching metrics of the at least two agents and the arbiter is adjusted by adjusting weights assigned to specific patient parameters and logical parsing rules specific to each agent upon multiple iterations.

The method finishes at step **S07.**

Pseudo code details of the exemplary algorithm as set out in a specific implementation of the above general steps of the method set out in flowchart of **FIG. 5****.** These steps for a specific example are described in steps 1-5 and set out in the Appendix hereto.

As described herein in an example, in step 1 transformer-based LLM agents (e.g., Gemma v3, DeepSeek R1, Llama 4) are selected based on their performance in understanding different clinical modalities (e.g. molecular biomarkers, disease, blood test results, demographics) in clinical trial criteria (i.e. inclusion/exclusion conditions). The performance of these agents are obtained from public leaderboards (e.g. Huggingface) and internal assessment using expert-curated dataset.

Relevant attributes a_c are extracted from patient P using a knowledge graph database (KGDB). The KGDB stores patients, clinical encounters, condition, treatment, and observation as nodes. Each condition, treatment, and observation node stores a link to relevant chunks of original clinical text of patient P, which are fed into LLM agents to assess if a clinical trial criterion c matches with the extracted patient attribute a_c. The LLM agents would also output a confidence value "conf"(a_c) for the match between c and a_c:

If the LLM agent thinks the extracted patient attribute is unreliable: $" conf " \left(a_c\right) = - 1$

If the LLM agent is uncertain if the patient attribute is reliable: $" conf " \left(a_c\right) = 0$

If the LLM agent thinks the extracted patient is reliable: $" conf " \left(a_c\right) = 1$

Next, in step 2, eligible trials are filtered using mandatory criteria. Mandatory criteria act as a filter. Only trials meeting all mandatory criteria are designated as Eligible Trials E. Currently, molecular biomarkers, primary diagnosis, gender and age are considered mandatory criteria. Other trial criteria are considered optional.

In step 3, trials are scored and ranked using Optional Criteria. This step assigns scores to eligible clinical trials based on a patient's profile P and ranks the Eligible Trials E to produce a list in descending order. For each eligible trial *Tᵢ*, the algorithm identifies the set of optional criteria, and calculated weighted sum of matching score *sᵢ*.

In step 4, Arbiter Resolution takes place. This step resolves discrepancies among clinical trials with similar scores within a similarity threshold (*∈*). The arbiter module incorporates contextual factors to adjust trial scores *S*[*Tₖ*]*.* Some contextual features (e.g. comorbidities) relies on a clinical-modality specific agent to re-assess the match function match(P, c). The ranked list R is then re-sorted based on updated scores. This step ensures precise rankings by resolving ties, prioritizing trials based on contextual patient-trial matching requirements, and allowing user-defined sorting criteria that may not be defined in clinical trial criteria.

The following hypothetical example illustrates how the contextual weights work.

### Patient parameter

Location: New York, USA (willing to travel up to 200km)
Comorbidities: Hypertension
Trials (*T*₁*, T*₂*, T*₃) with identical clinical scores (*sᵢ* = 0.8)
Phase priority: Phase I = 0.2, II = 0.5, III = 1.0
Context Weights (Initial): ${W}_{ctx} = \left\{{w}_{phase}=0.5,{w}_{location}=0.9,{w}_{comorb ,}=0.5,{w}_{urgency}=0.6,…\right\} ,$

| **Trial** | **Phase** | **Distance (km)** | **Comorbidity Match** | **Enrolment Urgency (days left / days of enrolment window)** |
|---|---|---|---|---|
| *T*₁ | III | 50 | Hypertension allowed | 30 / 365 |
| *T*₂ | II | 200 | Hypertension NOT allowed | 10/90 |
| *T*₃ | I | 50 | Hypertension allowed | 60 / 365 |

Normalize Contextual Features $\begin{matrix}{Δ}_{ctx} = \sum {w}_{contextual _ requirement} ⋅ normalisation _ factor \\ = {w}_{phase} ⋅ phase _ priority \left({T}_{k}\right) + {w}_{location} ⋅ \left(1-\frac{distance \left(P, {T}_{k}\right)}{max _ distance}\right) + {w}_{comorb} \\ ⋅ \left(\frac{matching _ comorbidities \left(P, {T}_{k}\right)}{total _ comorbidities}\right) + {w}_{urgency} ⋅ \left(1-\frac{days _ remaining}{total _ days}\right)\end{matrix}$

| | |
|---|---|
| For *T*₁: | |
| | Δ_{ctx} = 0.5 · 1.0 + 0.9 · 0.75 + 0.5 · 1.0 + 0.6 · 0.92 = 2.227 |
| For *T*₂ | |
| | Δ_{ctx} = 0.5 · 0.5 + 0.9 · 0 + 0.5 · 0 + 0.6 · 0.89 = 0.784 |
| For *T*₃: | |
| | Δ_{ctx} = 0.5 · 0.2 + 0.9· 0.75 + 0.5 · 1.0 + 0.6 · 0.84 = 1.779 |

Update scores and re-rank the trials
Original scores (*sᵢ* = 0.8) + adjustments:

| **Trial** | **Δ*_{ctx}*** | **New Score** | **Rank** |
|---|---|---|---|
| *T*₁ | +2.227 | 3.027 | 1 |
| *T*₂ | +0.784 | 1.584 | 3 |
| *T*₃ | +1.779 | 2.579 | 2 |

Result: Despite identical score *sᵢ*, *T*₁ ranks first due to Phase III + proximity + comorbidity accepted.

In step 5 weights are updated based on feedback.

Each weight is updated via regularized reward prediction error based on actual patient-trial enrolment outcome: ${w}_{f} ← {w}_{f} + α {{{e}^{- γ Δ t}}_{︸}}_{time decay} ⋅ \left({{{s}_{j}}_{︸}}_{actual}-{{σ \left({Δ}_{ctx}{T}_{k}\right)}_{︸}}_{predicted}\right) ⋅ {{factor _ contribution \left(f, {T}_{k}\right)}_{︸}}_{normalized feature value} - {λw}_{f}$

Where
- *α*:Learning rate (default = 0.1)
- *Δt*:Time elapsed since enrolment in months
- y:Time decay factor that controls how quickly older feedback is discounted (default = 0.01 for ~1% decay/month)
- *σ*(*x*):Sigmoid function to map predicted enrolment scores to [0,1]
- *λ*:L2 regularization term (default = 0.01), for overfitting mitigation.

The above embodiments are described by way of example only. Many variations are possible without departing from the scope of the disclosure as defined in the appended claims.

For clarity of explanation, in some instances the present technology may be presented as including individual functional blocks including functional blocks comprising devices, device components, steps or routines in a method embodied in software, or combinations of hardware and software.

To illustrate the potential efficacy of the disclosed system and method, simulated benchmark evaluations were conducted using a dataset of 183 synthetic patient profiles matched against 31,259 potential patient and clinical trial pairs from SIGIR, TREC 2021 and TREC 2022 datasets. Three configurations were compared: (A) a single-agent baseline using the state-of-the-art TrialGPT implementation; (B) the present invention including multi-agent evaluation, arbiter resolution, and iterative feedback-based weight refinement.

The performance benchmark with respect to other existing methods are summarized as follows. Table 1 is the performance of SciFive, BioBERT, PubMedBERT, SapBERT, BioLinkBERT, TrialGPT-Ranking fetched from the TrialGPT paper.

**Table 1**

| **Method Name** | **NDCG@10** |
|---|---|
| SciFive | 0.4271 |
| BioBERT | 0.4091 |
| PubMedBERT | 0.4327 |
| SapBERT | 0.4148 |
| BioLinkBERT | 0.4797 |
| TrialGPT-Ranking (GPT-3.5) | 0.5395 |
| TrialGPT-Ranking (GPT-4) | 0.7275 |
| Our invention | 0.7833 |

These results indicate that domain specialization across multiple agents in our invention improves matching accuracy by approximately 7.6% in NDCG@10 score. The arbiter resolution step was observed to most significantly improve rankings for trials where multiple criteria had similar weights, resolving tie-breaking scenarios that the weighted voting module alone could not differentiate. The feedback loop demonstrated progressive improvement over successive iterations, with NDCG@10 scores increasing from 0.6999 after the first iteration to 0.7833 after the fifty iterations as agent weights converged toward values reflecting expert clinical priorities.

It would be appreciated that these benchmark results are illustrative simulations intended to demonstrate the expected behavior of the system architecture, and that actual performance may vary depending on the quality of patient data, the specific LLMs employed, and the clinical domain. Therefore, we assessed the real-world performance of patient-trial matchin using a total of 22 studies spanning early-phase, phase 2, phase 3, expanded-access, profiling, and quality-of-life protocols in the Prince of Wales hospital (PWH). As a group, these studies reflect the practical complexity of contemporary oncology referral, in which eligibility depends on integrated review of molecular biomarkers, tumor histology, disease stage, prior treatment exposure, treatment resistance, and performance status, rather than diagnosis alone.

Table 2 shows the 22 trials included in the real-world study.

**Table 2**

| **Trial Name** | **CRE No.** | **CCTU code** | **ClinicalTrials.gov ID** |
|---|---|---|---|
| LOXO-RET-17001 | 2017.347-T | ACT011 | NCT03157128 |
| LIBRETTO-201_LOXO_EAP | 2019.457 | ACT015 | NCT03906331 |
| CLDK378A2X01B_Roll over study | 2019.453 | ACT016 | NCT02584933 |
| TPX-0005-01_Turning Point | 2019.541 | ACT017 | NCT03093116 |
| CJDQ443A12101_Novartis | 2020.626 | ACT030 | NCT05358249 |
| BA3011-001_BioAtla | 2019.040 | ACT031 | NCT03425279 |
| TAPISTRY_BO41932_Roche | 2021.540' | ACT034 | NCT04589845 |
| AMG193_20210023_AMGEN | 2022.341 | ACT038 | NCT05094336 |
| D35-001-100_D3 Bio | 2022.651 | ACT039 | NCT05410145 |
| BI1479-0001_BI | 2023.150' | ACT040 | NCT04886804 |
| ATORG-003_in house | 2020.020' | LUN094 | NCT04027647 |
| JSG-MC-JZJC-LIBRETTO-431_Lilly | 2020.056 | LUN096 | NCT04194944 |
| BAY2927088-BAY21607_BAYER | 2021.582 | LUN098 | NCT05099172 |
| CJDQ443B12301_Novartis | 2022.172 | LUN101 | NCT05132075 |
| TROPION-LUNG07 (DS1062-A-U303) | 2022.654 | LUN107 | NCT05555732 |
| TROPION-LUNG08 (DS1062-A-U304) | 2023.069 | LUN108 | NCT05215340 |
| ATORG molecular Profiling Project | 2018.375 | TRA018 | NA |
| ATORG-004 | 2019.523 | TRA019 | NCT04087473 |
| Brightline-1_BI_Liposarcoma | 2022.272 | SAR005 | NA |
| EORTC Sarcoma QOL tool development | 2020.022 | QOL014 | NA |
| DCC-3014-MOTION_Deciphera | 2022.074 | TGC001 | NCT05059262 |
| PEAK_GIST | 2023.038 | GIS001 | NCT05208047 |

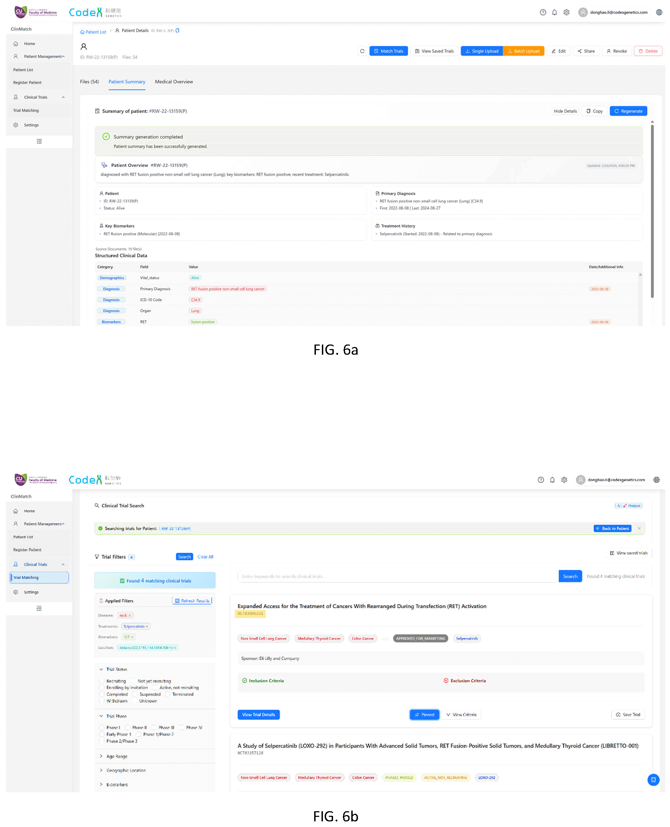

**FIG. 6a** to **FIG. 7b** depict examples of structured patient summary of two exemplary scenarios and exemplary identified matching results.

In both cases, scanned medical records were first transformed into structured patient summaries using the system as descripted above by implementing the method steps S00 to S07. The resulting summaries were then used to drive trial filtering and shortlist generation. These examples are provided as operational illustrations rather than a formal validation study, showing how the system can reduce the amount of manual interpretation required before investigator-level review.

### Case study 1: RET-altered lung cancer matched to ACT015

The first case concerns a patient subsequently enrolled in PWH's ACT015 study, Expanded Access for the Treatment of Cancers With Rearranged During Transfection (RET) Activation (NCT03906331). After uploaded documents were parsed, ClinMatch generated a structured summary that organized the record into diagnosis, biomarker, treatment-history, and supporting metadata fields. In this example, the summary captured a primary diagnosis of RET fusion-positive non-small cell lung cancer together with recent selpercatinib treatment and relevant location information. This restructuring is important because it converts free-text source material into trial-relevant variables that can be used directly in screening.

**FIG. 6a** presents an example of a structured patient summary generated from scanned medical records for the RET-altered lung cancer case. The interface surfaces diagnosis, key biomarker status, treatment history, and source-document traceability in a single review workspace.

Using these structured elements, the trial-matching interface returned four Hong Kong-relevant candidate studies, including the ACT015 trial in which the patient was ultimately enrolled. In operational terms, this case illustrates how ClinMatch narrows the search space by combining disease label, biomarker status, treatment context, and location filters rather than requiring staff to search manually across a larger portfolio of trials.

**FIG. 6b** presents trial-matching results for the same RET-altered case. Four candidate trials were returned for the Hong Kong setting, including ACT015 (NCT03906331), demonstrating how structured summaries can support targeted shortlisting.

### Case study 2: HER2/ERBB2-altered NSCLC matched within a broader candidate set

The second case concerns a patient enrolled in the Bayer 21607 study, a first-in-human trial of BAY2927088 in advanced non-small cell lung cancer with EGFR and/or HER2 alterations (NCT05099172). Here again, the workflow began with automated generation of a structured patient summary from uploaded records. The summary highlighted non-small cell lung cancer, an ERBB2 exon 20 insertion, and prior afatinib exposure, thereby organizing the clinically meaningful elements required for downstream trial filtering.

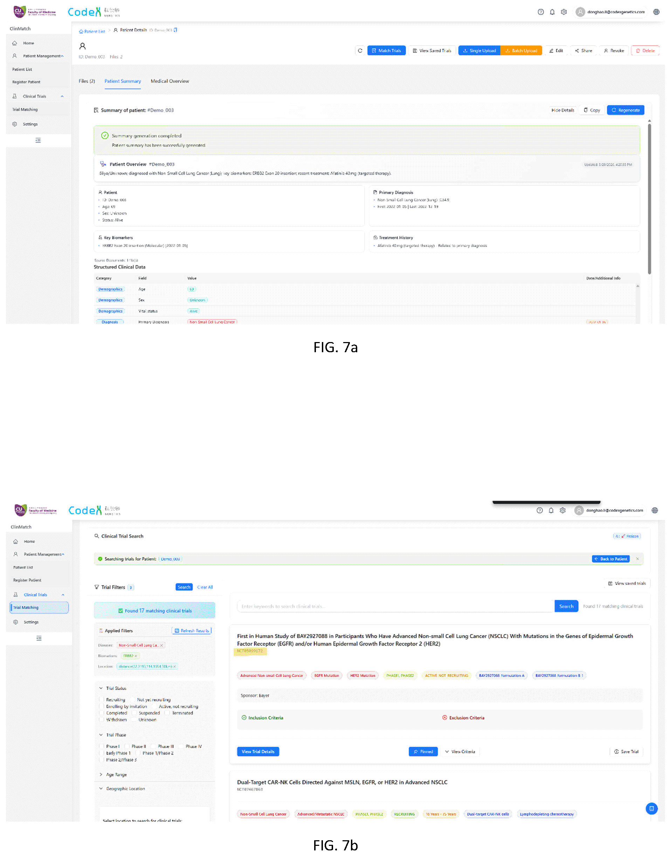

**FIG. 7a** illustrates a structured patient summary for the second. The summary condenses diagnosis, biomarker findings, and prior therapy into a trial-screening view.

In contrast to the first example, this case produced a broader set of 17 Hong Kong-matched studies. Even in a larger candidate set, however, the platform preserved the core logic needed for efficient screening by making diagnosis, biomarker, and location filters explicit and by surfacing the Bayer 21607 study within the shortlist. This case usefully illustrates optimization in a more complex matching context where the system does not eliminate the need for investigator review, but it substantially structures the first-pass identification process.

**FIG. 7b** shows trial-matching results for the HER2/ERBB2-altered NSCLC case. The shortlisted Hong Kong trials include the Bayer 21607 study (NCT05099172), demonstrating how the platform supports navigation through a larger pool of potentially relevant studies.

Taken together, these two examples show how ClinMatch optimizes the screening pathway by converting unstructured clinical records into structured, searchable summaries and then using those summaries to guide trial filtering.

Methods according to the above-described examples can be implemented using computer-executable instructions that are stored or otherwise available from computer readable media. Such instructions can comprise, for example, instructions and data which cause or otherwise configure a general purpose computer, special purpose computer, or special purpose processing device to perform a certain function or group of functions. Portions of computer resources used can be accessible over a network. The computer executable instructions may be, for example, binaries, intermediate format instructions such as assembly language, firmware, or source code. Examples of computer-readable media that may be used to store instructions, information used, and/or information created during methods according to described examples include magnetic or optical disks, flash memory, Universal Serial Bus (USB) devices provided with non-volatile memory, networked storage devices, and so on.

Devices implementing methods according to these disclosures can comprise hardware, firmware and/or software, and can take any of a variety of form factors. Typical examples of such form factors include laptops, smart phones, small form factor personal computers, personal digital assistants, and so on. Functionality described herein also can be embodied in peripherals or add-in cards. Such functionality can also be implemented on a circuit board among different chips or different processes executing in a single device, by way of further example.

The instructions, media for conveying such instructions, computing resources for executing them, and other structures for supporting such computing resources are means for providing the functions described in these disclosures.

Although a variety of examples and other information was used to explain aspects within the scope of the appended claims, no limitation of the claims should be implied based on particular features or arrangements in such examples, as one of ordinary skill would be able to use these examples to derive a wide variety of implementations. Further and although some subject matter may have been described in language specific to examples of structural features and/or method steps, it is to be understood that the subject matter defined in the appended claims is not necessarily limited to these described features or acts. For example, such functionality can be distributed differently or performed in components other than those identified herein. Rather, the described features and steps are disclosed as examples of components of systems and methods within the scope of the appended claims.

Appendix:

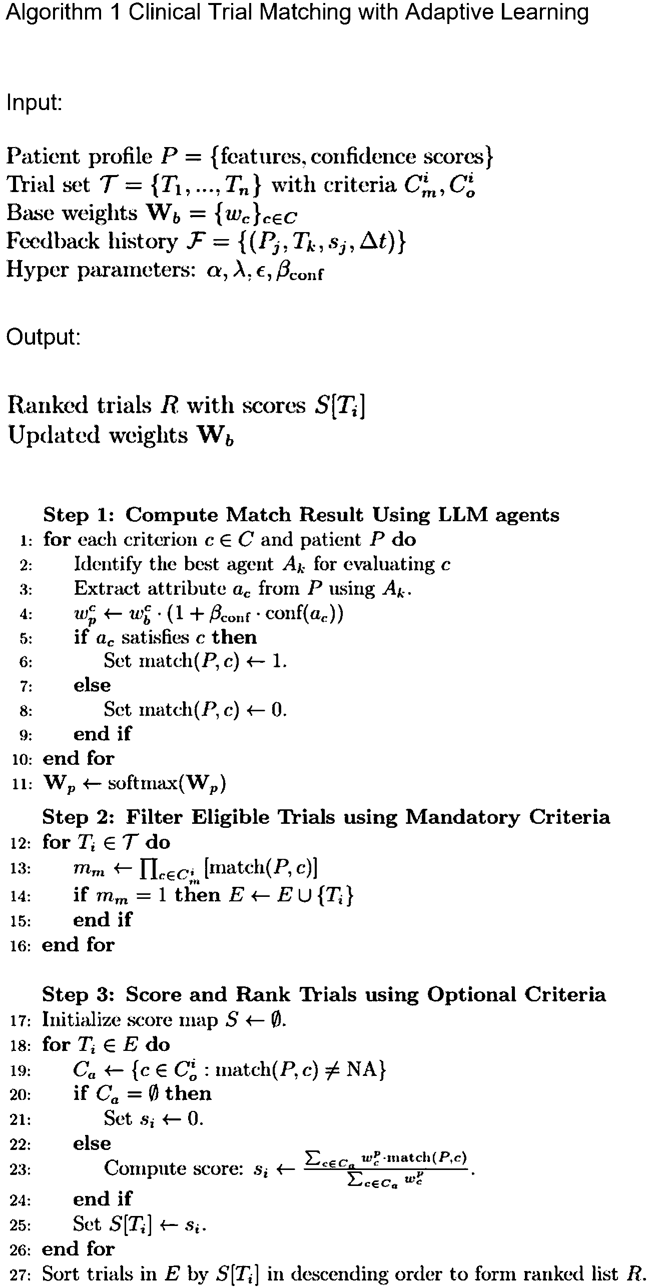

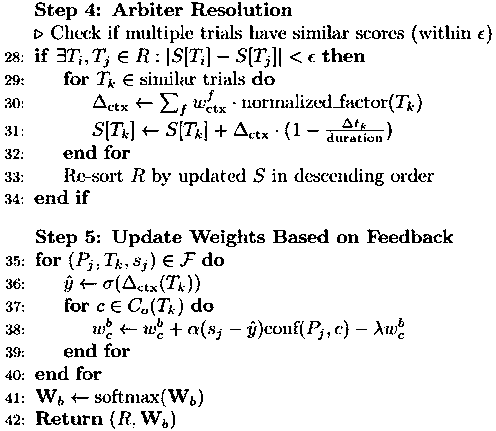

## Claims

1. A system for matching a subject with one or more suitable clinical trials from a plurality of clinical trials, comprising:
a receiving module configured to receive
(i) a patient profile of the subject, wherein the patient profile comprises a plurality of different parameters; and
(II) a plurality of clinical trials comprising clinical trial criteria with a plurality of different conditions;
an evaluation framework module for evaluating suitability of a patient profile for one or more of the plurality of clinical trials, the evaluation framework module comprising at least two evaluation agents, wherein each agent has a specific analysis domain different from the other; and each evaluation agent is configured to use a large language model;
and wherein each evaluation agent is configured to:
extract parameters from the patient profile and conditions of the clinical trial criteria according to the specific analysis domain of that agent and calculate a score indicative of the degree of match therebetween;
generate a ranked list of potential suitable clinical trials according to the score indicative of the degree of match for the specific analysis domain for that evaluation agent and the patient profile;
a consensus module configured to resolve discrepancies between the ranked trial lists for said patient profile by the at least two evaluation agents to determine a refined list comprising one or more suitable clinical trials by:
(a) using a weighted voting module if conflicting evaluations exist among the ranked lists of potential suitable clinical trials by prioritising trials with analysis domain results derived by more accurate evaluation agents to determine the refined list of suitable clinical trials for the subject; and
(b) if the score indicative of the degree of match of potential suitable clinical trials differs by a predetermined similarity difference, using an arbiter module configured to resolve conflicting evaluations, and generate the refined list of suitable clinical trials for the subject;
a feedback module configured to output the refined list of clinical trials for the patient profile and to receive data indicative of the suitability of the subject to a specific clinical trial.

2. The system of claim 1, further comprising a refinement module configured to dynamically adjust matching metrics of the at least two evaluation agents and the arbiter module based on the feedback data by adjusting weights assigned to specific patient parameters and logical parsing rules specific to each evaluation agent upon receiving data indicative of the suitability of the patient to a specific clinical trial.

3. The system of claim 1, wherein re-ranking by the arbiter module is performed by determining a custom probability score indicating suitability of at least some of the plurality of clinical trials for that subject.

4. The system of claim 1, wherein each evaluation agent is configured to cite supporting text for criteria used in the determination of the evaluation score and in the absence of supporting text, the evaluation agent is configured to exclude that data from the evaluation score until external verification.

5. The system of claim 1, wherein the arbiter module comprises a first arbiter module for resolving conflicting evaluations for specific contextual patient parameters and a second arbiter module comprises a large language model configured for resolving conflicting evaluations for specific non-contextual patient profile parameters.

6. The system of claim 1, wherein 0 is assigned as the score if the subject is evaluated as ineligible for the clinical trial and 1 is assigned as the score if the patient profile parameters and the conditions of the clinical trial criteria match perfectly or assigning a number between these values according to the degree of evaluated match.

7. The system of claim 1, further comprising a deidentification module configured to remove personally identifiable information from patient profiles by zero-knowledge proofs.

8. A computer implemented method for matching a subject with one or more suitable clinical trials from a plurality of clinical trials, the method comprising:
(i) receiving by one or more processors of a computer system and via a communication interface a patient profile of the subject comprising a plurality of different parameters and a plurality of clinical trials comprising clinical trial criteria with a plurality of different conditions;
(ii) using an evaluation framework module to evaluate suitability of a patient profile for one or more of the plurality of clinical trials, wherein the evaluation framework comprises at least two evaluation agents executing on the one or more processors; wherein each evaluation agent is configured to have a specific analysis domain different from the other evaluation agent, wherein each evaluation agent is configured to use a large language model and wherein each evaluation agent is configured to:
extract parameters from the patient profile and conditions of the clinical trial criteria according to the specific analysis domain of that agent and calculate a score indicative of the degree of match therebetween; and
generate a ranked list of potential suitable clinical trials according to the score indicative of the degree of match for the specific analysis domain for that evaluation agent and the patient profile;
(iii) resolving by a consensus module executing on the one or more processors discrepancies between the ranked trial lists for said patient profile generated by the at least two evaluation agents to determine a refined list comprising one or more suitable clinical trials by:
(a) using a weighted voting module if conflicting evaluations exist among the ranked lists of potential suitable clinical trials by prioritising trials with analysis domain results derived by more accurate evaluation agents where the accuracy of the evaluation agent for a specific domain is stored in the memory of the computer system to determine the refined list of suitable clinical trials for the subject; and
(b) if the score indicative of the degree of match of potential suitable clinical trials differs by a predetermined similarity difference, using an arbiter module configured to resolve conflicting evaluations and stored in the memory of the computer system for generating the refined list of suitable clinical trials for the subject;
(iv) outputting the refined list of clinical trials for the patent profile via a user interface or device and receiving data indicative of the suitability of the subject to a specific clinical trial.

9. The method of claim 8, further comprising dynamically adjusting matching metrics of the at least two evaluation agents and the arbiter module based on the feedback data by adjusting weights assigned to specific patient parameters and logical parsing rules specific to each agent upon receiving data indicative of the suitability of the patient to a specific clinical trial.

10. The method of claim 8, wherein re-ranking by the arbiter module is performed by determining a custom probability score indicating suitability of at least some of the plurality of clinical trials for that subject.

11. The method of claim 8, wherein each evaluation agent is configured to cite supporting text for criteria used in the determination of the evaluation score and in the absence of supporting text, the evaluation agent is configured to exclude that data from the evaluation score until external verification.

12. The method of claim 8, wherein the arbiter module comprises a first arbiter module configured for resolving conflicting evaluations for specific contextual patient parameters and a second arbiter module comprises a large language model configured for resolving conflicting evaluations for specific non-contextual patient parameters.

13. The method of claim 8, wherein 0 is assigned as the score if the subject is evaluated as ineligible for the clinical trial and 1 is assigned as the score if the patient profile parameters and the conditions of the clinical trial criteria match perfectly or assigning a number between these values according to the degree of evaluated match.

14. The method of claim 8, further comprising removing personally identifiable information from patient profiles by zero-knowledge proofs.

15. A non-transitory machine-readable medium for matching a subject with one or more suitable clinical trials of a plurality of clinical trials, the non-transitory machine-readable medium storing instructions which, when executed by one or more processors of a computing system, causes the one or more processors to perform operations comprising the steps of any one of claims 8 to 14.
